# FASCICULE DE BREVET EUROPEEN

(11) **EP 2 117 356 B1**
(45) Date de publication et mention de la délivrance du brevet: **04.07.2012**
(21) Numéro de dépôt: 07731500.0
(22) Date de dépôt: 17.01.2007
(51) Int. Cl.: A23L 1/302, A61K 45/06, A23L 1/30, A61K 36/23, A61K 36/82, A61K 36/87, A61K 36/81, A61K 36/45, A23L 1/303

(54) **COMPOSITION DIETETIQUE ANTIOXYDANTE A BASE DE FRUITS ET LEGUMES, SON PROCEDE D'OBTENTION ET UTILISATION DE LA COMPOSITION**
ANTIOXIDANS-NAHRUNGSMITTELZUSAMMENSETZUNG, DIE FRÜCHTE UND GEMÜSE ENTHÄLT, VERFAHREN ZU IHRER HERSTELLUNG UND VERWENDUNG DER ZUSAMMENSETZUNG
ANTI-OXIDANT DIETARY COMPOSITION CONTAINING FRUITS AND VEGETABLES, METHOD FOR PREPARING THE SAME AND USE OF THE COMPOSITION

(43) Date de publication de la demande: 18.11.2009
(73) Titulaire: Fytexia, 34350 Vendres (FR)
(72) Inventeur: DALLAS, Constantin, F-34500 Beziers (FR)
(74) Mandataire: Rhein, Alain
(86) Numéro de dépôt international: PCT/FR2007/050657
(87) Numéro de publication internationale: WO 2008/090291

(56) Documents cités:
- WO-A-01/89542
- WO-A-02/100329
- DE-U1- 20 316 876
- US-A- 5 904 924
- US-A- 6 054 128
- US-A1- 2005 048 143
- US-A1- 2005 250 703
- DATABASE EPODOC EUROPEAN PATENT OFFICE, THE HAGUE, NL; 10 août 2006 (2006-08-10), LASKIN VUL F. A., BALJURA A.V. [RU]: "foodstuff kit for weekly diet ..." XP002452846 & RU 105 668 A 10 août 2006 (2006-08-10)
- DATABASE EPODOC EUROPEAN PATENT OFFICE, THE HAGUE, NL; 14 décembre 1993 (1993-12-14), KANEKO TOSHIRO: "Tea tablet" XP002453130 -& JP 05 328947 A 14 décembre 1993 (1993-12-14)

## Description

L'invention concerne une composition antioxydante à usage diététique, en particulier obtenue à partir de fruits et légumes.

L'invention concerne encore un procédé pour l'obtention d'une telle composition ainsi que l'utilisation d'une telle composition.

L'invention entre dans le domaine des compléments alimentaires.

Il est connu dans le domaine de la diététique l'intérêt d'une consommation appropriée de fruits et légumes frais pour les bienfaits qu'elle procure à l'organisme.

On considère ainsi qu'un régime alimentaire équilibré doit comprendre au moins cinq fruits et légumes différents par jour, correspondant environ à 400 grammes en poids de fruits et légumes frais.

Du fait des contraintes de vie moderne, notamment citadine, atteindre un tel objectif est le plus souvent aléatoire.

Il est un objet de la présente invention d'apporter une solution pour atteindre cet objectif.

De plus, les choix alimentaires de la consommation moderne encouragent le plus souvent une prise importante de graisses ou lipides au détriment d'autres éléments nutritifs tout aussi essentiels, les conséquences pouvant aller d'une prise de poids déraisonnée, jusque, par exemple, au déclanchement de maladies cardio-vasculaires causées par l'accumulation de lipides à l'intérieur des artères.

Il est ainsi un autre objet de la présente invention d'apporter une solution de prévention du développement de maladies cardio-vasculaires.

Il est encore connu qu'une alimentation déséquilibrée, conduisant à des défaillances nutritionnelles voire des carences, est une source de stress oxydatif pour les organismes vivants. Ce stress oxydatif peut encore être associé à notre environnement de vie (pollution, tabac, U.V., etc.) ou provenir de ces divers facteurs (alimentation et/ou environnement de vie) pris ensemble. Ce stress oxydatif est dû à un excès d'espèces réactives oxygénées, comme les anions superoxydes, le peroxyde d'hydrogène ou les radicaux hydroxyles, dans les cellules de l'organisme par rapport aux capacités qu'ont ces cellules de les maîtriser.

Ce stress oxydatif est également en partie à l'origine des effets du vieillissement, notamment cutané.

Il est connu des compositions à usage diététique tentant de pallier cet inconvénient, comme décrites dans les documents US 5 904 924, US 6 054 128 ou bien dans l'abrégé XP 002452846 issu de la base de données EPODOC de l'Office Européen des Brevets. De telles compositions sont issues de mélanges d'espèces végétales avec une teneur en polyphénols, conférant une capacité antioxydante correspondant à au moins 5000 micromoles/gramme de Trolox équivalents. Ces compositions sont souvent utilisées en tant que complément alimentaire.

Toutefois, de telles compositions n'offrent pas un résultat optimal et des qualités oxydantes satisfaisante.

Il est encore un autre objet de l'invention que de proposer une solution anti-âge.

L'invention se propose de suppléer à ces multiples inconvénients conduisant à une alimentation mal équilibrée ou en résultant, en proposant un complément alimentaire particulièrement conçu pour son action à la fois antioxydante et ciblée pour prévenir les risques de maladies cardio-vasculaires.

A cet effet, l'invention concerne une composition diététique, comprenant au moins des polyphénols et des caroténoïdes, lesdits polyphénols étant représentés par au moins 1 à 30% de procyanidines, 5 à 50% de flavanols et 0,1 à 10% d'anthocyanosides, les pourcentages se référant à la composition totale et le total de polyphénols présents atteignant au moins 60% en poids de la composition totale, caractérisée en ce qu'elle est obtenue à partir d'un mélange d'espèces végétales comportant au moins 15 à 25% en poids de raisins rouges et/ou blancs (*Vitis vinifera*), 5 à 10% en poids de myrtilles (*Vaccinium myrtillus*), 10 à 20% en poids de tomates (*Solanum lycopersicum*), 10 à 20% en poids de carottes (*Daucus carota*), et 10 à 20% en poids de thé vert (*Camelia sinensis*), ladite composition comprenant 0,1 à 2% en poids de vitamine C, 0,1 à 1,5% en poids de vitamine B et 0,1 à 2% en poids de caroténoïdes, lesdits caroténoïdes étant présentés par au moins du lycopéne et/ou du beta-carotène.

La composition diététique selon l'invention comprend en plus de la vitamine C et des vitamines de type B.

Les polyphénols sont présents dans la composition pour au moins 60% en poids, les caroténoïdes pour 0,1 à 2% en poids, la vitamine C pour 0,1 à 2% en poids et les vitamines de type B pour 0,1 à 1,5% en poids.

En outre, cette composition diététique peut être obtenue à partir d'un mélange comprenant en plus une ou plusieurs espèces végétales parmi l'orange (*Citrus aurantium dulcis*), le pamplemousse (*Citrus grandis*), la papaye (*Carica papaya*), l'ananas (*Ananas sativus*), la fraise (*Fragaria vesca*), la pomme (*Purus malus*), l'abricot (*Prunus armeniaca*), la cerise (*Prunus* avium), le cassis (*Ribes nigrum*), le brocolis (*Brassica oleracea*), le chou vert (*Brassica oleracea*), l'oignion (*Allium cepa*), l'ail (*Allium sativum*), l'olive (*Olea europaea*), les germes de blé (germes de *Triticum vulgare*), le concombre (*Cucumis sativus*) et l'asperge (*Asparagus officinalis*).

De préférence, la composition diététique se présente, seule ou avec des adjuvants supplémentaires, sous forme d'une poudre ou d'une poudre hydrosoluble.

La composition diététique se présente avantageusement sous forme d'une poudre ayant une capacité antioxydante correspondant à au moins 5000 µmoles/gramme de Trolox équivalents.

L'invention concerne encore un procédé pour l'obtention d'une telle composition.

Un tel procédé comporte les étapes suivantes :
- d'extraction des substances actives par macération d'un broyat obtenu à partir d'un mélange d'espèces végétales comportant au moins 15 à 25% en poids de raisins rouges et/ou blancs (*Vitis vinifera*), 5 à 10% en poids de myrtilles (*Vaccinium myrtillus*), 10 à 20% en poids de tomates (*Solanum lycopersicum*), 10 à 20% en poids de carottes (*Daucus carota),* et 10 à 20% en poids de thé vert (*Camelia sinensis*);
- de centrifugation du macérat obtenu
- de concentration des substances actives; et
- de séchage par atomisation ;

Il se caractérise en ce que l'on procède à l'étape de séchage par atomisation en partant d'une phase liquide, contenant les substances actives, ayant une capacité antioxydante entre environ 15000 et 25000 µmoles/gramme de Trolox équivalents.

L'invention concerne encore l'utilisation de la composition comme complément alimentaire et/ou pour enrichir des aliments, notamment les boissons telles que les jus de fruits et/ou légumes ainsi que les produits lactés.

L'invention concerne encore l'utilisation de la composition pour son administration diététique, notamment suivant une posologie entre 10 et 25 mg, préférentiellement environ 21,5 mg, de composition par kilogramme de poids corporel par jour.

L'invention concerne encore l'utilisation pour son administration diététique en vue de diminuer le taux de cholestérol total, en particulier le taux de cholestérol lié aux protéines à basse densité (LDL), et/ou l'index athérogénique, et/ou augmenter les capacités antioxydantes du plasma sanguin.

L'invention concerne encore l'utilisation pour son administration diététique en vue de réduire la quantité de radicaux libres présents dans l'organisme, en particulier par la réduction de la production d'anion superoxyde, et/ou réduire les dépôts de plaques lipidiques aortiques.

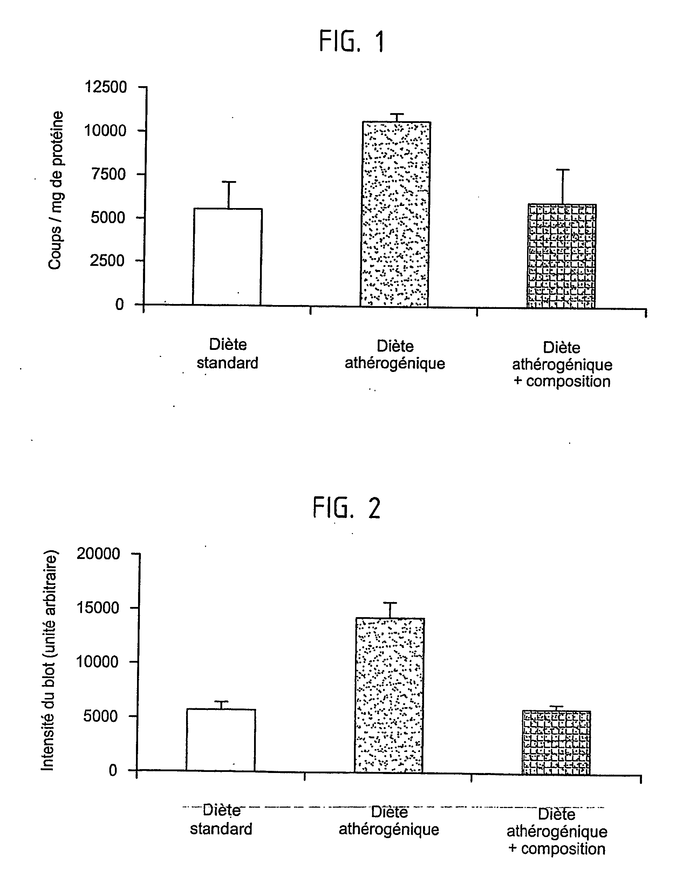

Les buts et avantages de la présente invention apparaîtront à description détaillée qui va suivre se rapportant à plusieurs exemples de réalisation. La compréhension de cette description sera facilitée en se référant aux dessins ci-joint, dans lesquels les figures 1 à 3 présentent des résultats expérimentaux attestant l'efficacité de la composition selon l'invention.

La présente invention concerne le domaine des compléments alimentaires et a trait, plus particulièrement, à une composition antioxydante à usage diététique, en particulier obtenue à partir de fruits et légumes.

Les espèces végétales pour son obtention ont été retenues sur la base de leurs apports nutritionnels bénéfiques sur la santé, de manière à obtenir une composition apportant un juste dosage de substances actives agissant en synergie.

En particulier et selon un mode particulier de réalisation, la composition selon l'invention tire parti d'une synergie entre les effets des polyphénols et des caroténoïdes qu'elle contient.

Avantageusement et selon un mode préférentiel de réalisation, la synergie est augmentée par l'association en plus de vitamine C et de plusieurs types de vitamines B administrés en une seule prise.

Les polyphénols constituent un groupe de substances chimiques retrouvées dans les plantes, et ayant des propriétés antioxydantes. La composition selon l'invention présente plus précisément des teneurs particulières en procyanidines, en flavanols et en anthocyanosides.

Les caroténoïdes sont essentiellement présents sous forme de lycopène et/ou de beta-carotène.

Le tableau 1 présente le dosage de ces différentes substances actives essentielles dans un mode de réalisation préféré de la composition selon l'invention.

La composition diététique selon l'invention comprend, en poids, au moins 60% de polyphénols et 0,1 à 2% de caroténoïdes.

Les polyphénols sont représentés par des procyanidines, des flavanols et des anthocyanosides.

La composition comprend plus particulièrement, en poids, 1 à 30% de procyanidines, 5 à 50% de flavanols et 0,1 à 10% d'anthocyanosides.

Les caroténoïdes ont une représentation en lycopène et/ou en béta-carotène équivalente à 0,1 à 1% en poids de la composition finale pour chaque molécule.

La vitamine C est présente, en poids, à hauteur de 0,1 à 2% et les vitamines B à hauteur de 0,1 à 1,5%.

Les vitamines de type B font partie du groupe constitué par la vitamine B1, la vitamine B6 et la vitamine PP, seules ou selon toutes combinaisons entre les constituants de ce groupe, et avec des teneurs dans la composition d'environ 0,1 à 0,5% pour les vitamines B1 et B6, et d'environ 0,1 à 1% pour la vitamine PP.

Les pourcentages sont calculés à partir de l'analyse d'un échantillon de composition au travers de diverses méthodes. Les polyphénols totaux sont dosés à l'aide de la spectrophotométrie Ultra-Violet à 280 nm, et les anthocyanosides à l'aide de la même technique à 520 nm. Les caroténoïdes totaux sont également dosés par spectrophotométrie Ultra-Violet. Les procyanidines, les flavanols, la vitamine C et les vitamines B sont quant à eux dosés par Chromatographie Liquide Haute Performance (HPLC).

Un procédé optimisé que nous décrirons par ensuite permet d'obtenir la composition diététique selon l'invention, qui se présente au final sous forme de poudre, standardisée en capacité antioxydante. Avantageusement 1 gramme de composition selon l'invention permet de délivrer au moins 5000 valeurs ORAC (Oxygen Radical Absorbance Capacity), ce qui revient à dire que la composition finale en poudre contient au moins 5000 µmoles/gramme de Trolox équivalents, qui est également une mesure conventionnelle du pouvoir oxydant d'une substance.

Ce pouvoir oxydant calibré à 5000 valeurs ORAC par gramme correspond à un cahier des charges réfléchi pour l'élaboration de la composition et son procédé d'obtention selon l'invention. En effet, il est ressorti d'études sur le pouvoir oxydant des fruits et légumes que cinq fruits et légumes d'un poids total de 400 grammes contiennent en moyenne environ 4000 valeurs ORAC. Une composition délivrant 5000 valeurs ORAC par gramme de composition permet, en admininistrant 0,8 grammes de composition, d'atteindre les 4000 valeurs ORAC contenues dans une consommation de 400 grammes de fruits et légumes frais. De la même manière, l'administration de 1,6 grammes de composition selon l'invention permettra d'arriver aux 8000 valeurs ORAC que contiennent 800 grammes de fruits et légumes, généralement atteints par la consommation de 10 fruits et légumes par jour.

De façon surprenante, des expérimentations ont reconnu le dosage en substances actives présenté plus haut comme particulièrement efficace pour répondre aux objectifs diététiques visés, à savoir un effet anti-âge et une réduction des risques de maladies cardiovasculaires.

Pour une meilleure compréhension de ces expérimentations, il est nécessaire d'exposer au préalable les facteurs biologiques en jeu dans certaines maladies cardiovasculaires comme l'athérome, qui est une des étiologies dominante de la majorité des affections cardiovasculaires.

L'athérome correspond à un remaniement de l'intima des artères de gros et moyen calibre suite à l'accumulation segmentaire de lipides, glucides complexes, sang et produits sanguins, tissus adipeux et dépôts calcaires.

En particulier, il est connu que le développement de l'athérome est initié par l'oxydation de lipoprotéines à basse densité (LDL). Cette oxydation est elle-même favorisée par un stress oxydatif important, qu'il soit d'origine alimentaire et/ou environnementale.

A ce propos, les lipoprotéines à basse densité (LDL) transportent le cholestérol des lieux de sécrétion vers les cellules de l'organisme.

En effet, le cholestérol étant un composé hydrophobe, il n'est pas soluble dans le sang et son transport est assuré par des lipoprotéines auxquelles il se fixe.

Des taux importants de lipoprotéines à basse densité (LDL) conduisent généralement au dépôt de cholestérol sur les parois des artères sous forme de plaque d'athérome, ce qui accroît le risque de maladies cardiovasculaires et leur vaut le nom de « mauvais cholestérol ».

Les lipoprotéines à haute densité (HDL), déchargent quant à elles les artères et les tissus extrahépatiques du cholestérol, en le transportant vers le foie où il est dégradé. On parle généralement de « bon » cholestérol en faisant référence à ces lipoprotéines.

Les causes du stress oxydatif favorisant l'athérome ne sont pas bien connues mais de récents travaux ont montré qu'une des sources majeures de génération d'espèces réactives oxygénées, qui sont à l'origine du stress oxydatif, est constituée par l'activité d'une enzyme, la NAD(P)H oxydase.

Cette enzyme associée aux membranes est composée de 5 sous-unités, et catalyse la réduction de l'oxygène, en utilisant le NADP ou NADPH comme donneur d'électron. La NAD(P)H oxydase génère de quantités significatives de radicaux superoxydes et une association entre son activité enzymatique et les facteurs de risques cliniques d'athérome ont été démontrées. Azumi et al. (Expression of NADH/NADPH oxidase p22phox in human coronary arteries. Circulation 1999, 100, 1494-8) ont montré que la sévérité des lésions apparaissant dans l'athérome étaient corrélées avec la surexpression de la sous-unité p22phox, constitutive de la NAD(P)H oxydase, dans les artères coronaires.

En fait, il semble qu'un cycle amplificateur est amorcé en cas d'athérome, qui constitue une situation pathologique, où les unités membranaires de la NAD(P)H oxydase, comme l'unité p22phox, sont sur-exprimées. La NAD(P)H oxydase a ainsi un rôle reconnu dans la pathogenèse de l'athérome.

Pour en revenir aux expérimentations, celles-ci ont été conduites sur des Hamsters dorés, encore appelés Hamsters de Syrie, mâles, d'un poids variant entre 85 et 95 grammes.

Le choix des hamsters pour la réalisation de ces expériences est justifié par le fait que les hamsters ont une distribution de lipoprotéines plasmatiques similaire à celle retrouvée chez les humains et que les transporteurs majeurs du cholestérol plasmatique sont les lipoprotéines à basse densité (LDL).

Un certain nombre de ces animaux ont été soumis à une diète qualifiée d'athérogénique, c'est-à-dire destinée à favoriser l'apparition d'athérome, car riche en cholestérol et en graisses. De manière à induire un stress peroxydatif, cette diète a également été rendue déficiente en vitamines C et E ainsi qu'en sélénium.

Egalement, une composition selon l'invention diluée dans de l'eau a été administrée, selon des modalités précisées plus loin dans le descriptif des expériences, à un certain nombre de ces animaux. Une analyse de la composition utilisée dans ces expériences a été préalablement faite et est résumée dans le tableau 2.

Les expérimentations menées peuvent être résumées en trois expériences révélatrices des effets physiologiques d'une administration de la composition selon l'invention.

### EXPERIENCE 1

Une première expérience dont les résultats figurent en tableau 4 visait la détection des effets d'une administration de la composition selon l'invention sur les concentrations plasmatiques en lipides et la capacité antioxydante du plasma sanguin chez les hamsters soumis à une diète athérogénique.

Cette expérience a été menée sur deux groupes de 18 hamsters chacun, chaque groupe ayant une moyenne équivalente en termes de poids corporels. Les hamsters de chacun de ces deux groupes ont été nourris pendant 84 jours suivant une diète athérogénique.

Cette diète athérogénique consiste en l'administration de 200g/kg de poids corporel de caséine, 3g/kg de L-méthionine, 393g/kg d'amidon de maïs, 154g/kg de sucrose, 50g/kg de cellulose, 150g/kg de saindoux, 5g/kg de cholestérol, un mélange de minéraux à hauteur 35g/kg et un mélange de vitamines à hauteur de 10mg/kg. De plus, le mélange de vitamines ne comprend ni sélénium, ni vitamines E et C.

Les hamsters ont également été hydratés de force soit en utilisant de l'eau du robinet pour le premier groupe, dit groupe de contrôle, soit par une composition selon l'invention diluée dans de l'eau pour le second groupe, dit groupe expérimental. Le volume des solutions administrées ont été ajustées quotidiennement au poids de chaque animal, selon la règle de volume de 7,14 mL par kilogramme corporel. Les hamsters du groupe expérimental ont reçu une dose de 21,4 mg par kilogramme corporel de la composition selon l'invention, dissoute dans un volume d'eau calculé selon la règle précédemment mentionnée. Cette dose de 21,4 mg de composition par kilogramme de poids corporel procède d'une corrélation faite avec une consommation chez l'homme d'environ 800 grammes de fruits et légumes frais par jour. Comme vu précédemment, la valeur ORAC de cette consommation est atteinte par l'administration de 1,6 grammes de composition selon l'invention. En considérant que le consommateur humain moyen pèse environ 70 kg, on arrive à reproduire, chez les hamsters, l'ingestion d'environ 10 fruits et légumes par jour, d'un poids d'environ 800 grammes, en leur administrant cette dose de 21,4 mg de composition par kilogramme de poids corporel quotidiennement.

A l'issue de ce traitement, le taux de cholestérol total et le taux de cholestérol lié aux lipoprotéines à haute densité (HDL) a été déterminé en utilisant des méthodes enzymatiques commerciales.

La capacité antioxydante du plasma a été mesurée en équivalents Trolox, qui est une mesure quantitative du niveau général en antioxydants dans les échantillons biologiques. La technique, conventionnelle, est colorimétrique et illustre la capacité d'un échantillon à éliminer un radical cationique coloré.

Il n'est pas inutile de préciser qu'il n'a été constaté aucune différence entre les masses corporelles initiales, les masses corporelles finales, et la quantité de nourriture prise entre les deux groupes, comme résumé dans le tableau 3.

Ainsi que le montrent les résultats figurant dans le tableau 4, l'administration de la composition selon l'invention a permis la réduction du taux de cholestérol plasmatique total de 15% et du taux de cholestérol non lié aux lipoprotéines à haute densité (HDL), donc essentiellement le taux de cholestérol lié aux lipoprotéines à basse densité (LDL), de 33% par rapport au groupe de contrôle. Le taux de cholestérol lié aux lipoprotéines à haute densité (HDL) n'a pas été modifié. Par conséquent, l'index athérogénique, correspondant au ratio entre le cholestérol total et le cholestérol lié aux lipoprotéines à haute densité (HDL) a été abaissé de 12,3% chez les hamsters ayant reçu la composition antioxydante selon l'invention.

De plus, l'administration de la composition selon l'invention a permis un renforcement des capacités antioxydantes du plasma sanguin, améliorant ces capacités de 10%.

En conclusion, l'administration de la composition selon l'invention améliore le profil lipidique du plasma sanguin et renforce ses capacités antioxydantes.

### EXPERIENCE 2

Une seconde expérience dont les résultats se trouvent illustrés en figures 1 et 2 visait la détection de la production en anion superoxyde, agent du stress oxydatif produit majoritairement par la NAD(P)H oxydase, et l'évolution de l'expression de la NAD(P)H oxydase lors d'un traitement avec la composition selon l'invention.

Cette expérience a été faite en parallèle sur des hamsters nourris avec une diète standard, des hamsters nourris avec une diète athérogénique et une administration de force d'eau du robinet, et des hamsters nourris avec une diète athérogénique et une administration d'eau additionnée de la composition selon l'invention.

La diète standard consiste en l'administration de 200g/kg de poids corporel de caséine, 3g/kg de L-méthionine, 447g/kg d'amidon de maïs, 175g/kg de sucrose, 50g/kg de cellulose, 80g/kg d'huiles végétales, un mélange de minéraux à hauteur 35g/kg et un mélange de vitamines à hauteur de 10mg/kg.

Les trois groupes de hamsters, composés chacun de 6 individus, ont été nourris pendant 84 jours suivant les mêmes modalités que celles de l'expérience 1.

A l'issue de ce traitement, la détermination de la production en anion superoxyde a été évaluée par une technique conventionnelle de détection de l'anion superoxyde par chemiluminescence. Les ventricules gauches des hamsters ont été placés dans une solution tampon contenant 250 Molaires de lucigénine et l'intensité de luminescence résultante a été mesurée avec un luminomètre.

Les résultats de cette opération, réalisée sur les trois groupes contenant chacun 6 hamsters, sont illustrés par le graphique de la figure 1. Ce graphique présente en ordonnée la mesure, moyenne sur tous les hamsters d'un groupe, de chemiluminescence en coups par mg de protéine, cette valeur étant d'autant plus élevée que la présence d'anion superoxyde est importante. En abscisse figurent les trois groupes de hamsters observés.

On a ensuite procédé à l'extraction des protéines contenues dans les ventricules gauches congelés des mêmes hamsters pour procéder, de manière conventionnelle, à l'immunoblotting de la sous-unité p22phox, afin de mesurer les différences d'expression en NAD(P)H oxydase dans chacun des 3 groupes de hamsters.

Les résultats présentés sur le graphique de la figure 2 sont issus d'un logiciel de traitement de données d'immunoblotting après acquisition de l'image du gel obtenu, et montrent la présence relative de la sous-unité p22phox par une mesure de l'intensité des blots obtenus sur le gel. En ordonnée figure l'intensité du blot correspondant à la sous-unité p22phox sur le gel, en unité arbitraire. En abscisse figurent les trois groupes de hamsters observés.

Il ressort de ces résultats que la production d'anion superoxyde (Figure 1) et l'expression de la sous-unité p22phox (Figure 2) ont respectivement diminués de 45,5% et 59,1% chez les hamsters ayant reçus la composition selon l'invention par rapport aux hamsters sous diète athérogénique ne l'ayant pas reçue.

### EXPERIENCE 3

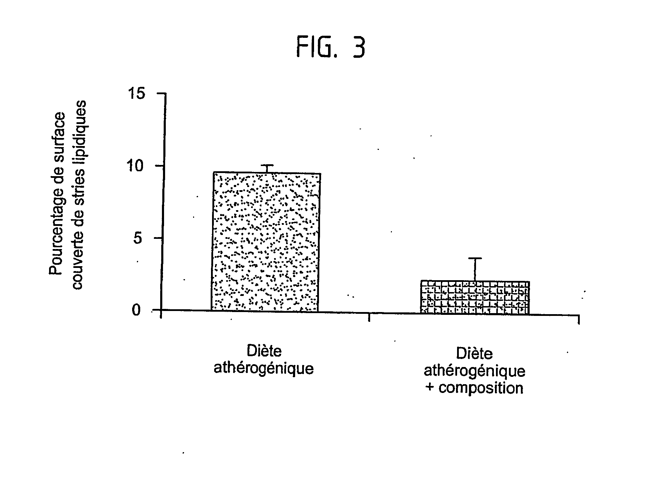

Une troisième expérience dont les résultats se trouvent illustrés en figure 3 a cherché la visualisation, au niveau de l'aorte des hamsters, de l'étendue des stries lipidiques.

En effet, l'athérome débute par des infiltrations lipidiques, appelées stries lipidiques, au niveau de l'intima, entraînant un épaississement de cet intima. On assiste ensuite à une prolifération de cellules musculaires lisses et de tissu conjonctif, entraînant la formation d'une plaque instable inflammatoire.

Cette expérience a été faite en parallèle sur des hamsters nourris avec une diète standard, des hamsters nourris avec une diète athérogénique et une administration de force d'eau du robinet, et des hamsters nourris avec une diète athérogénique et une administration d'eau additionnée de la composition selon l'invention.

Les trois groupes de hamsters, composés chacun de 12 individus, ont été nourris pendant 84 jours suivant les mêmes modalités que celles des expériences 1 et 2.

A l'issue de ce traitement, les animaux ont été sacrifiés, et après avoir collecté leur sang et retiré leur foie, leur aorte a été perfusée avec une solution pour la fixation de leur vascularisation et disséquée entre les valves sigmoïdes et 3-4 cm après l'arche aortique. La partie aortique extraite a ensuite été nettoyée, coupée et ouverte longitudinalement, puis plongée dans une solution fixante. Après rinçages, les segments aortiques ont été disposés sur un verre pour préparation microscopique, le côté endothélial vers le haut, et montés sur un microscope. Après photographie et numérisation, les surfaces de l'intima présentant des stries lipidiques ont été exprimées en pourcentage du total des surfaces examinées.

Le graphique de la figure 3 présente en ordonnée ces pourcentages moyennés dans chaque groupe de hamsters observé, l'étendue des stries lipidiques augmentant avec la valeur du pourcentage. En abscisse figurent les deux groupes de hamsters où des stries lipidiques ont été observées.

Il est à noter qu'aucune strie lipidique n'a été détectée chez les hamsters soumis à la diète standard. L'accumulation moyenne de stries lipidiques aortiques a été significativement réduite de 77% chez les hamsters ayant reçus la composition selon l'invention par rapport aux hamsters sous diète athérogénique ne l'ayant pas reçue.

Ceci montre l'effet bénéfique de la composition selon l'invention pour la réduction effective des facteurs macroscopiques précurseurs des maladies cardiovasculaires.

Afin d'obtenir la composition selon l'invention, il a été procédé à une sélection de fruits et légumes dont la combinaison est particulièrement adaptée pour l'obtention de ladite composition. Le tableau 5 présente les espèces végétales essentielles nécessaires, selon un mode particulier de réalisation, où la composition est obtenue à partir d'un mélange de raisin (rouge et blanc), de myrtille, de tomate, de carotte et de thé vert. Avantageusement, un pourcentage en poids fixant la représentation de chaque espèce végétale est respecté.

Le tableau 6 présente un mode préférentiel de réalisation, à partir d'un mélange de 22 fruits, légumes et autres espèces végétales différentes.

Enfin, la composition selon l'invention est obtenue grâce à un procédé préférentiel, dont les étapes sont retranscrites ci-dessous.

Particulièrement au niveau de la récolte et le transport des matières premières ainsi que pendant la phase d'extraction, il convient de protéger au maximum les substances actives, notamment les polyphénols, d'une oxydation. On pourra à ces fins appliquer des stratégies comme le travail sous atmosphère inerte, en particulier pendant l'extraction.

Une première étape consiste en un broyage des matières premières. Les espèces végétales sélectionnées sont broyées, individuellement ou combinées en différents groupes, dans un broyeur, à un ou plusieurs couteaux, à grande vitesse. Toutes les parties alimentaires des espèces végétales, notamment les fruits et légumes, peuvent être employées, à savoir la peau, le coeur, le jus, les graines ou encore les feuilles. Les couteaux sont entraînés en rotation à une vitesse de 6000 à 12000 révolutions par minute et pendant une durée de 2 à 5 minutes.

Une deuxième étape consiste en l'extraction des substances actives du broyat par macération. L'extraction peut être réalisée à l'eau préalablement amenée à une température de 50 à 90°C ou aux solvants organiques, par exemple l'éthanol ou l'acétate d'éthyle, qui sont compatibles avec un usage dans l'industrie alimentaire. De manière préférée, il est réalisé une double extraction en utilisant un mélange d'eau et de solvants organiques dans des proportions de 70 à 30 % d'eau et respectivement 30 à 70 % de solvants organiques.

Le processus d'extraction est réalisé dans une cuve en acier inoxydable et sous atmosphère inerte pour éviter l'oxydation des molécules actives, à une température entre 40 et 90°C et sous agitation durant 5 à 20 heures.

Divers paramètres influent sur le temps de macération, tels la concentration en substances actives des produits de départ, la finesse de broyage obtenue, la température d'extraction ou encore les solvants utilisés.

D'autres opérations conventionnelles pourront être conduites pour une telle extraction, comme des macérations successives, une extraction sous reflux ou encore sous pression réduite.

Une troisième étape consiste en la centrifugation du macérat. Après un repos sans agitation à température ambiante pendant 10 à 20 heures, le macérat est centrifugé afin d'en séparer toutes les particules solides, et récupérer la phase liquide contenant les substances actives extraites.

Une quatrième étape consiste en la concentration des substances actives. La phase liquide obtenue est concentrée par distillation ou évaporation. Cette étape dure entre 5 et 15 heures. Le distillat ou le résidu d'évaporation re-solubilisé, contenant les substances actives extraites, est alors soumis à une mesure de sa valeur en ORAC, selon des techniques conventionnelles.

D'un point de vue qualitatif, la valeur ORAC de la phase liquide requise à ce stade doit être située entre 15000 et 25000 µmole/gramme de Trolox équivalents.

On peut également adjoindre des étapes de purifications supplémentaires. L'extrait concentré peut ainsi être soumis à d'autres opérations conventionnelles de purification comme une filtration sur membrane de cellulose et/ou une décoloration.

Une dernière étape consiste sécher par atomisation l'extrait concentré résultant des opérations précédentes. Celui-ci est finalement mélangé à une matrice, préférentiellement de la maltodextrine compatible avec un usage alimentaire, et pulvérisé pour séchage par des techniques conventionnelles de manière à obtenir une fine poudre, contenant au minimum environ 5000 µmoles/gramme de Trolox équivalents (valeur ORAC) et 50 à 90% de polyphénols.

Au travers du procédé selon l'invention, il est ainsi possible d'obtenir une composition répondant avantageusement au problème posé, à savoir permettant de rétablir un apport diététique quotidien en fruits et légumes, d'agir en prévention des maladies-cardiovasculaires et lutter contre les effets généraux du vieillissement par sa capacité anti-oxydante.

Avantageusement, la poudre obtenue à l'issue du procédé selon l'invention est hydrosoluble.

L'invention concerne encore l'utilisation de la composition obtenue comme complément alimentaire et/ou pour enrichir des aliments.

De manière non exhaustive, la poudre obtenue peut être employée, seule ou avec des adjuvants supplémentaires, sous forme galénique de poudre libre, de poudre encapsulée afin notamment d'éviter son oxydation, par exemple en gélule, de poudre compressée en comprimés ou granulés de toutes formes, ou encore ensachée.

La poudre obtenue peut encore être employée, seule ou avec des adjuvants supplémentaires, par exemple en étant dissoute dans un liquide alimentaire, et ainsi incorporée à des boissons, ou bien dans des produits lactés tels des yaourts.

De manière non exhaustive, les boissons peuvent par exemple être des jus de fruits et/ou légumes.

L'invention concerne encore l'utilisation de la composition obtenue pour son administration diététique.

Finalement, on rappellera que l'administration de la composition selon l'invention permet avantageusement la diminution du taux de cholestérol total, et en particulier le taux de cholestérol lié aux lipoprotéines à basse densité (LDL), et/ou la diminution de l'index athérogénique, et/ou l'augmentation des capacités antioxydantes du plasma sanguin.

L'administration de la composition selon l'invention permet aussi la réduction de la quantité de radicaux libres présents dans l'organisme, en particulier par la réduction de la production d'anion superoxyde et/ou la réduction de dépôts de plaques lipidiques aortiques.

La posologie préférentielle se situe entre 0,5 et 0,8 grammes par jour de composition lorsque utilisée comme complément alimentaire et 0,5 à 0,8 grammes par litre de composition lorsque utilisée dans des boissons. Cette posologie peut être adaptée pour correspondre à une administration entre 10 et 25 mg, préférentiellement environ 21,5 mg, de composition par kilogramme de poids corporel par jour. En effet, 10 mg/kg corporel correspondent environ a l'apport en valeurs ORAC d'une consommation de 5 fruits et légumes frais, et 21,5 mg/kg corporel à l'apport de 10 fruits et légumes frais, la posologie pouvant être modulée suivant les apports recherchés.

**TABLEAU 1 : Substances Actives essentielles**

| Substances actives | | % de la composition |
|---|---|---|
| 1) Polyphénols totaux | | 60-100% |
| | Procyanidines (dimères, trimères) | 1-30% |
| | Flavanols (monomères) | 5-50% |
| | Anthocyanosides | 0,1-10% |
| 2) Caroténoïdes totaux | | 0,1-2% |
| | Lycopène | 0,1-1% |
| | Beta-carotène | 0,1-1% |
| 3) Vitamine C | | 0,1-2% |
| 4) Vitamines de type B | | 0,1-1,5% |
| | Vitamine B1 (Thiamine) | 0,1-0,5% |
| | Vitamine B6 (Pyridoxine) | 0,1-0,5% |
| | Vitamine PP (Niacine) | 0,1-1% |

**TABLEAU 2 : Substances Actives détectées dans la composition selon l'invention utilisée pour les expériences**

| Substances actives | | Teneurs |
|---|---|---|
| 1) Polyphénols | | |
| | Dimères de Procyanidines B1, B2, B3 et B4 | 1,14 g/100g |
| | Monomères de flavanols sous forme de catéchines monomériques : | |
| | - catéchine | 0,55 g/100g |
| | - épicatéchine | 3,08 g/100g |
| | - èpicatèchine-3-O-gallate | 4,10 g/100g |
| | - épigallocathéchine | 4,17 g/100g |
| | - épigallocathéchine-3-O-gallate | 21,33 g/100g |
| | Anthocyanosides | 0,6 g/100g |
| | Acide gallique | 0,15 g/100g |
| 2) Caroténoïdes | | |
| | Lycopène | 28 mg/100g |
| 3) Vitamine C | | 4,92 mg/100g |

**TABLEAU 3 : Effets de l'administration de la composition selon l'invention sur les concentrations plasmatiques en lipides et la capacité antioxydante du plasma sanguin chez des hamsters soumis à une diète athérogénique.**

| | Groupe de contrôle | Groupe Expérimental |
|---|---|---|
| Poids initial (g) | 91.3±2.1 | 86.7±5.8 |
| Poids final (g) | 130.9±9.7 | 129.6±1.8 |
| Prise de nourriture (g/jour) | 3.47±0.90 | 3.49±0.60 |

**TABLEAU 4 : Effets de l'administration de la composition selon l'invention sur les concentrations plasmatiques en lipides et la capacité antioxydante du plasma sanguin chez des hamsters soumis à une diète athérogénique.**

| | Groupe de contrôle | Groupe Expérimental |
|---|---|---|
| Cholestérol total (mmol/L) | 5.92±0.17 | 5.00±0.12 |
| Cholestérol lié aux lipoprotéines à haute densité (mmol/L) | 3.81±0.14 | 3.60±0.09 |
| Cholestérol non lié aux lipoprotéines à haute densité (mmol/L) | 2.18±0.23 | 1.40±0.13 |
| Index athérogénique | 1.59±0.34 | 1.40±0.18 |
| Capacité antioxydante du plasma (mmol/L) | 1.29±0.06 | 1.42±0.10 |

**TABLEAU 5 : Espèces végétales essentielles**

| Espèces végétales de la composition | | Nom botanique | % de la composition |
|---|---|---|---|
| Extraits de fruits | | | |
| | Raisin (rouge et blanc) | *Vitis vinifera* | 15-25% |
| | Myrtille | *Vaccinium myrtillus* | 5-10% |

| Autres extraits | | | |
|---|---|---|---|
| | Tomate | *Solanum lycopersicum* | 10-20% |
| | Carotte | *Daucus carota* | 10-20% |
| | Thé vert | *Camelia sinensis* | 10-20% |

**TABLEAU 6 : Liste exhaustive des espèces végétales pouvant entrer dans une composition selon l'invention**

| Espèces végétales de la composition | | Nom botanique |
|---|---|---|
| Extraits de fruits | | |
| | Raisin (rouge et blanc) | *Vitis vinifera* |
| | Myrtille | *Vaccinium myrtillus* |
| | Orange | *Citrus aurantium dulcis* |
| | Pamplemousse | *Citrus grandis* |
| | Papaye | *Carica papaya* |
| | Ananas | *Ananas sativus* |
| | Fraise | *Fragaria vesca* |
| | Pomme | *Purus malus* |
| | Abricot | *Prunus armeniaca* |
| | Cerise | *Prunus avium* |
| | Cassis | *Ribes nigrum* |
| Autres extraits | | |
| | Tomate | *Solanum lycopersicum* |
| | Carotte | *Daucus carota* |
| | Thé vert | *Camelia sinensis* |
| | Brocoli | *Brassica oleracea* |
| | Chou vert | *Brassica oleracea* |
| | Oignon | *Allium cepa* |
| | Ail | *Allium sativum* |
| | Olive | *Olea europaea* |
| | Germes de blé | *germes de Triticum vulgare* |
| | Concombre | *Cucumis sativus* |
| | Asperge | *Asparagus officinalis* |

## Revendications

1. Composition diététique, compressant au moins des polyphénols et des caroténoïdes, lesdits polyphénols étant représentés par au moins 1 à 30% de procyanidines, 5 à 50% de flavanols et 0,1 à 10% d'anthocyanosides, les pourcentages se référant à la composition totale et la total de polyphénols présents atteignant au moins 60% en poids de la composition totale, **caractérisée en ce qu'**elle est obtenue à partir d'un mélange d'espèces végétales comportant au moins 15 à 25% en poids de raisins rouges et/ou blancs (*Vitis vinifera*), 5 à 10% en poids de myrtilles (*Vaccinium myrtillus*), 10 à 20% en poids de tomates (*Solanum lycopersicum*), 10 à 20% en poids de carottes (*Daucus* carota), et 10 à 20% en poids de thé vert (*Camelia sinensis*), ladite composition comprenant 0,1 à 2% en poids de vitamine C, 0,1 à 1,5%, en poids de vitamine B et 0 ,1 à 2% en poids de caroténoïdes, lesdits caroténoïdes étant représentés par au moins du lycopène et/ou du beta-carotène.

2. Composition diététique selon la revendication 1, **caractérisée en ce qu'**elle est obtenue à partir d'un mélange comprenant en plus une ou plusieurs espèces végétales parmi l'orange (*Citrus aurantium dulcis*), le pamplemousse (*Citrus grandis*), la papaye (*Carica papaya*), l'ananas (*Ananas sativus*), la fraise (*Fragaria ve*s*ca*), la pomme (*Purus malus*), l'abricot (*Prunus armeniaca*), la cerise (*Prunus avium*), le cassis (*Ribes nigrum*), le brocolis (*Brassica oleracea*), le chou vert (*Brassica oleracea*), l'oignion (*Allium cepa*), l'ail (*Allium sativum*), l'olive (*Olea europaea*), les germes de blé (germes de *Triticum vulgare*), le concombre (*Cucumis sativus*) et l'asperge (*Asparagus officinalis*).

3. Composition diététique selon l'une des revendications 1 ou 2, **caractérisée en ce qu'**elle se présente, seule ou avec des adjuvants supplémentaires, sous forme d'une poudre ou d'une poudre hydrosoluble.

4. Composition diététique selon la revendication 3, **caractérisée en ce qu'**elle se présente sous forme d'une poudre ayant une capacité antioxydante correspondant à au moins 5000 µmoles/gramme de Trolox équivalents.

5. Procédé pour l'obtention d'une composition diététique selon les revendications 1 à 4, comportant des étapes :
- d'extraction des substances actives par macération d'un broyat obtenu à partir d'un mélange d'espèces végétales comportant au moins 15 à 25% en poids de raisins rouges et/ou blancs (*Vitis vinifera*), 5 à 10% en poids de myrtilles (*Vaccinium myrtillus*), 10 à 20% en poids de tomates (*Solanum lycopersicum*), 10 à 20% en poids de carottes (*Daucuns carota*), et 10 à 20% en poids de thé vert (*Camelia sinensis*) ;
- de centrifugation du macérat obtenu
- de concentration des substances actives ; et
- de séchage par atomisation ;
**caractérisé en ce que** l'on procède à l'étape de séchage par atomisation en partant d'une phase liquide, contenant les substances actives, ayant une capacité antioxydants entre environ 15000 et 25000 µmoles/gramme de Trolox équivalents.

6. Procédé selon la revendication 5 **caractérisé en ce que** l'extraction des substances actives est obtenu à partir d'un mélange comprenant en plus une ou plusieurs espèces végétales parmi l'orange (*Citrus aurantium dulcis*), le pamplemousse (*Citrus grandis*), la papaye (*Carica papaya*), l'ananas (*Ananas sativus*), la fraise (*Fragaria vesca*), la pomme (*Purus malus*), l'abricot (*Prunus azmeniaca*), la cerise (*Prunus avium*), le cassis (*Ribes nigrum*), le brocolis (*Brassica oleracea*), le chou vert (*Brassica oleracea*), l'oignon (*Allium cepa*), l'ail (*Allium sativum*), l'olive (*Olea europaea*), les germes de blé (germes de *Triticum vulgare*) le concombre (*Cucumis sativus*) et l'asperge (*Asparagus officinalis*).

7. Utilisation de la composition, diététique selon les revendications 1 à 4 pour son administration diététique suivant une posologie entre 10 et 25 mg, préférentiellement environ 21,5 mg, de composition diététique par kilogramme de poids corporel par jour.

8. Utilisation de la composition diététique selon les revendications 1 à 4 comme complément alimentaire.

9. Utilisation de la composition diététique selon les revendications 1 à 4 pour enrichir les aliments, notamment les boissons telles que les jus de fruits et/ou légumes ainsi que les produits lactés.

10. Utilisation de la composition diététique selon les revendications 1 à 4, pour son administration diététique en vue de diminuer le taux de cholestérol total, en particulier le taux de cholestérol lié aux protéines à basse densité (LDL), et/ou l'index athérogénique, et/ou augmenter les capacités antioxydantes du plasma sanguin.

11. Utilisation de la composition diététique selon les revendications 1 à 4, pour son administration diététique, en vue de réduire la quantité de radicaux libres présents dans l'organisme, en particulier par la réduction de la production d'anion superoxyde, et/ou réduire les dépôts de plaques lipidiques aortiques.

## Claims

1. Dietetic composition, comprising at least polyphenols and carotenoids, said polyphenols being represented by at least 1 to 30% of procyanidins, 5 to 50% of flavonols and 0.1 to 10% of anthocyanins, the percentages referring to the total composition and the total of polyphenols present reaching at least 60 wt % of the total composition, wherein it obtained from a mixture of plant species comprising at least 15 to 25 wt % of red and/or white grapes (*Vitis vinifera*), 5 to 10 wt % of blueberries (*Vaccinium myrtillus*), 10 to 20 wt % of tomatoes (*Solanum lycopersicum*), 10 to 20 wt % of carrots (*Daucus carota*), and to 20 wt % of green (*Camellia sinensis*), said composition comprising 0.1 to 2 wt % of vitamin C, 0.1 to 1.5 wt % of vitamine B and 0.1 to 2 wt % of carotenoids, said carotenoids being represented by at least lycopens and/or beta-carotene.

2. Dietetic composition according to claim 1, wherein it is obtained from a mixture comprising in addition one or several plant species among orange (*Citrus aurantium dulcis*), grapefruit (*Citrus grandis*), papaya (*Carica papaya*), pineapple (*Ananas sativus*), strawberry (*Fragaria vesca*), apple (*Pyrus malus*), apricot (*Prunus armeniaca*), cherry *(Prunus avium),* blackcurrant (*Ribes nigrum*), broccoli (*Brassica oleracea*), curly kale *(Brassica oleracea),* onion (*Allium cepa*), garlic (*Allium sativum*), olive *(Olea europaea),* wheat germs (germs of *Triticum vulgare*), cucumber (*Cucuvis sativus*) and asparagus (*Asparagus officinalis*).

3. Dietetic composition according to one of claims 1 or 2, wherein it is, alone or with additional additives, in the form of a powder or water-soluble powder.

4. Dietetic composition according to claim 3, wherein it is in the form of a powder having an antioxidant capacity corresponding to at least 5000 µmol/gram equivalent of Trolox.

5. A method for obtaining the dietetic composition according claims 1 to 4, comprising the steps of:
- extracting active substances by maceration of a ground material obtained from a mixture of plant species comprising at least 15 to 25 wt % of red and/or white grapes (*Vitis vinifera*), 5 to 10 wt % of blueberries (*Vaccinium myrtillus*), 10 to 20 wt % of tomatoes (*Solanum lycopersicum*), 10 to 20 wt % of carrots *(Daucus carota),* and 10 to 20 wt % of green tea (*Camellia sinensis*);
- centrifuging the macerate obtained;
- concentration of active substances; and
- spray-drying;
wherein one proceeds to the step of spray-drying starting from a liquid phase containing the active substances, having an antioxidant capacity between about 15,000 and 25,000 µmol/gram equivalent of Trolox.

6. A method according to claim 5, wherein the extraction of the active substances is obtained from a mixture comprising in addition one or several plant species among orange (*Citrus aurantium dulcis*), grapefruit (*Citrus grandis*), papaya *(Carica papaya),* pineapple (*Ananas sativus*), *strawberry* (*Fragaria vesca*), apple (*Pyrus malus*), apricot (*Prunus armeniaca*), cherry *(Prunus avium),* blackcurrant (*Ribes nigrum*), broccoli *(Brassica oleracea),* curly kale *(Brassica oleracea),* onion (*Allium cepa*), garlic (*Allium sativum*), olive (*Olea europaea*), wheat germs (germs of *triticum vulgare*), cucumber (*Cucuvis sativus*) and asparagus (*Asparagus officinalis*).

7. Usage of the dietetic composition according to claims 1 to 4 for its dietetic administration in a dosage between 10 and 25 mg, preferably of about 21.5 mg, of dietetic composition per kilogram of body weight per day.

8. Usage of the dietetic composition according to claims 1 to 4 as a food supplement.

9. Usage of the dietetic composition according to claims 1 to 4 for enriching foods, namely drinks such as fruit and/or vegetable juices and dairy products.

10. Usage of the dietetic composition according to claims 1 to 4 for its dietetic administration in order to reduce the total choresterol rate, in particular the low-density protein-related (LDL) cholesterol rate, and/or the atherogenic index, and/or to increase the antioxidant capacity of blood plasma.

11. Usage of the dietetic composition according to claims 1 to 4 for its dietetic administration in order to reduce the quantity of free radicals present in the body, in particular by reducing the superoxide anion production, and/or to reduce the deposits of aortic lipid plaques.

## Patentansprüche

1. Diätetische Nahrungsmittelzusammensetzung, die zumindest Polyphenole und Carotenoide enthält, wobei die besagten Polyphenole durch zumindest 1 bis 30% Procyanidine, 5 bis 50% Flavanole und 0,1 bis 10% Anthocyanoside repräsentiert sind, wobei sich die Prozentanteile auf die Gesamtzusammensetzung beziehen, und der Gesamtanteil der vorhandenen Polyphenole zumindest 60 Gewichts-% der Gesamtzusammensetzung erreicht, **dadurch gekennzeichnet, dass** sie aus einer Pflanzenartenmischung hergestellt ist, die zumindest 15 bis 25 Gewichtes-% rote und/oder weiße Traubenbeeren (*Vitis vinifera*), 5 bis 10 Gewichts-% Heidelbeeren (*Vaccinium myrtillus*), 10 bis 20 Gewichts-% Tomaten (*Solanum lycopersicum*), 10 bis 20 Gewichts-% Mohrrüben *(Daucus carota)* und 10 bis 20 Gewichts-% Teepflanze *(Camellia sinensis)* umfasst, wobei die besagte Nahrungsmittelzusammensetzung 0,1 bis 2 Gewichts-% Vitamin C, 0,1 bis 1,5 Gewichts-% Vitamin B und 0,1 bis 2 Gewichts-% Carotenoide enthält, wobei die besagten Carotenoide zumindest durch Lykopin und/oder Beta-Karotin repräsentiert sind.

2. Diätetische Nahrungsmittelzusammensetzung nach Anspruch 1, **dadurch gekennzeichnet, dass** sie aus einer Mischung hergestellt ist, die außerdem eine oder mehrere unter nachfolgend aufgeführten Pflanzenarten enthält: Orange *(Citrus aurantium dulcis)*, Pampelmuse *(Citrus grandis),* Papayafrucht *(Carica papaya),* Ananas *(Ananas sativus),* Erdbeere *(Fragaria vesca),* Apfel *(Purus malus),* Aprikose *((Prunus armeniaca),* Vogelkirsche *(Prunus avium),* schwarze Johannisbeere *(Ribes nigrum),* Broccoli *(Brassica oleracea), Grünkohl (Brassica oleracea),* Zwiebellauch *(Allium cepa),* Knoblauch *(Allium sativum),* Olive *(Olea europaea),* Weizenkeime *(Keime von Triticum vulgare),* Gurke *(Cucumis sativus)* und Gemüsespargel *(Asparagus officinalis).*

3. Diätetische Nahrungsmittelzusammensetzung nach einem der Ansprüche 1 oder 2, **dadurch gekennzeichnet, dass** sie allein oder zusammen mit zusätzlichen Hilfsstoffen in Form von einem Pulver oder von einem wasserlöslichen Pulver vorliegt.

4. Diätetische Nahrungsmittelzusammensetzung nach Anspruch 3, **dadurch gekennzeichnet, dass** sie in Form von einem Pulver vorliegt, das eine antioxidative Kapazität, die zumindest 5000 Mikromol Trolox-Äquivalenten pro Gramm entspricht, aufweist.

5. Verfahren zur Herstellung einer diätetischen Nahrungsmittelzusammensetzung nach Ansprüchen 1 bis 4, umfassend folgende Schritte:
- Extraktion der wirksamen Substanzen durch die Mazeration eines zerkleinerten Pflanzenguts, das aus einer Mischung von Pflanzenarten aufbereitet ist, die zumindest 15 bis 25 Gewichts-% rote und/oder weiße Traubenbeeren *(Vitis vinifera),* 5 bis 10 Gewichts-% Heidelbeeren *(Vaccinium myrtillus),* 10 bis 20 Gewichts-% Tomaten *(Solanum lycopersicum),* 10 bis 20 Gewichts-% Mohrrüben *(Daucus carota)* und 10 bis 20 Gewichts-% Teepflanze *(Camellia sinensis)* umfasst;
- Zentrifugieren des erhaltenen Mazerats;
- Konzentration der wirksamen Substanzen; und
- Sprühtrocknung,
**dadurch gekennzeichnet, dass** der Schritt zur Sprühtrocknung startend von einer flüssigen Phase erfolgt, die die wirksamen Substanzen enthält, die eine antioxidative Kapazität zwischen etwa 15000 und 25000 Mikromol Trolox-Äquivalenten pro Gramm aufweisen.

6. Verfahren nach Anspruch 5, **dadurch gekennzeichnet, dass** die Extraktion der wirksamen Substanzen aus einer Mischung erfolgt, die außerdem eine oder mehrere unter nachfolgend aufgeführten Pflanzenarten enthält: Orange *(Citrus aurantium dulcis),* Pampelmuse *(Citrus grandis),* Papayafrucht *(Garica papaya),* Ananas *(Ananas sativus),* Erdbeere *(Fragaria vesca),* Apfel *(Purus malus)*, Aprikose *(Prunus armeniaca),* Vogelkirsche *(Prunus avium),* schwarze Johannisbeere *(Ribes nigrum),* Broccoli *(Brassica oleracea),* Grünkohl *(Brassica oleracea),* Zwiebellauch *(Allium* cepa), Knoblauch *(Allium sativum),* Olive *(Olea europaea),* Weizenkeime *(Keime von Triticum vulgare),* Gurke *(Cucumis sativus*) und Gemüsespargel *(Asparagus officinalis)*.

7. Verwendung der diätetischen Nahrungsmittelzusammensetzung nach den Ansprüchen 1 bis 4 für ihre diätetische Verabreichung nach einer Dosierung zwischen 10 und 25 mg, vorzugsweise etwa 21,5 mg von der diätetischen Nahrungsmittelzusammensetzung pro Kilogramm Körpergewicht pro Tag.

8. Verwendung der diätetischen Nahrungsmittelzusammensetzung nach Ansprüchen 1 bis 4 als Ergänzungslebensmittel.

9. Verwendung der diätetischen Nahrungsmittelzusammensetzung nach Ansprüchen 1 bis 4, um Nahrungsmittel, insbesondere Getränke wie Obst- und/oder Gemüsesäfte sowie Milchprodukte anzureichern.

10. Verwendung der diätetischen Nahrungsmittelzusammensetzung nach Ansprüchen 1 bis 4 für ihre diätetische Verabreichung, um den Gesamt-Cholesterinspiegel, insbesondere den LDL-Cholesterinspiegel, der mit den Proteinen geringer Dichte zusammenhängt, und/oder den atherogenen Index abzusenken und/oder die antioxidative Kapazität des Blutplasmas zu erhöhten.

11. Verwendung der diätetischen Nahrungsmittelzusammensetzung nach Ansprüchen 1 bis 4 für ihre diätetische Verabreichung, um die Menge der im Körper vorhandenen freien Radikale insbesondere durch die Reduzierung der Bildung von Hyperoxidanionen herabzusetzen und/oder die lipidischen Ablagemngsplaques in der Aorta zu reduzieren.
